# EUROPÄISCHE PATENTSCHRIFT

(11) **EP 2 366 365 B1**
(45) Veröffentlichungstag und Bekanntmachung des Hinweises auf die Patenterteilung: **24.04.2013**
(21) Anmeldenummer: 11156771.5
(22) Anmeldetag: 03.03.2011
(51) Int. Cl.: A61F 2/50

(54) **Erwärmungsvorrichtung für einen thermoplastischen Prothesenschaftrohling**
Heating device for a thermoplastic prosthetic shank blank
Dispositif de chauffage pour une ébauche de prothèse thermoplastique

(30) Priorität: 17.03.2010 DE 102010011799
(43) Veröffentlichungstag der Anmeldung: 21.09.2011
(73) Patentinhaber: medi GmbH & Co. KG, 95448 Bayreuth (DE)
(72) Erfinder: Kremser, Dirk, 23863 Bargfeld - Stegen (DE); Kurth, Christof, 95500, Heinersreuth (DE)
(74) Vertreter: Blaumeier, Jörg

(56) Entgegenhaltungen:
- WO-A1-2008/092617
- DE-A1- 10 026 399
- US-A- 5 376 129

## Beschreibung

Die Erfindung betrifft eine Erwärmungsvorrichtung für einen thermoplastischen Prothesenschaftrohling umfassend ein Gestell, eine damit verbundene Halterung für den Prothesenschaftrohling sowie eine Heizeinrichtung. Das Dokument WO 2008/092617A1 stellt der nächte Stand der Technik dar.

Zur Herstellung eines Prothesenschaftes ist es in der Regel nötig, ein Amputationsstumpfmodell, ein sogenanntes Positiv, herzustellen. Hierzu wird der Stumpf z. B. mit einer Gipsbinde in der gewünschten Form und Stellung abgeformt. Anschließend wird mithilfe dieses Abdrucks, also der Negativform, wieder ein Positivmodell erstellt. Das erfolgt z. B. durch Ausgießen mit Gips oder Hartschaum. Das so entstandene Amputationsstumpfmodell wird dann noch in geeigneter Weise nachgearbeitet. Eine andere Möglichkeit besteht darin, den Amputationsstumpf zu scannen oder diverse diskrete Maße zu nehmen und diese Daten in eine geeignete Bildbearbeitungs- oder Konstruktionssoftware zu überführen Auf diese Weise entsteht ein digitales Modell, welches rechnergestützt nachgearbeitet werden kann. Anschließend wird auf Basis der nachgearbeiteten CAD-Daten beispielsweise mit Hilfe einer Fräse maschinell das Amputationsstumpfmodell erstellt.

Das so hergestellte Positiv umhüllend wird der Prothesenschaft gefertigt. Dabei wird zwischen Definitiv- und Testschaft unterschieden. Der Testschaft wird in der Regel aus einem thermoplastischen Kunststoff gefertigt, ist zumeist transparent und kann nachträglich thermoplastisch verändert werden. Die Haltbarkeit ist allerdings begrenzt; bei Schlagbeanspruchung kann es zur spontanen Ausbildung scharfkantiger Risse im Schaft kommen. Daher wird nach einer Testschaftversorgung und deren Passformoptimierung ein Definitivschaft hergestellt. Dieser weist eine höhere Stabilität, Langlebigkeit und Bruchfestigkeit auf. Er wird zumeist aus faserverstärktem Laminat hergestellt. Auf diese Weise hergestellte Definitivschäfte sind nicht transparent und nicht nachformbar.

Die Herstellung eines transparenten Testschafts erfolgt in der Regel durch Tiefziehen einer bis zu 15 mm dicken Kunststoffplatte. Diese wird in einem Tiefziehrahmen eingespannt und zunächst in einem Umluftofen erwärmt, bis sie sich plastisch verformt und im Tiefziehrahmen durchhängt, wobei die Erwärmung solange erfolgt, bis die Platte einen Durchhang von ca. 20 - 30 cm aufweist. Anschließend wird der Rahmen samt Platte aus dem Ofen genommen, um 180° gedreht und über das Positiv gezogen. Anfänglich wird das erwärmte Kunststoffmaterial mechanisch über die Positivlänge des Stumpfmodells gezogen, anschließend wird zur Anformung an das Modell ein Unterdruck zwischen dem Positiv und dem Thermoplast angelegt, wodurch das erweichte Material an das nachbearbeitete Amputationsstumpfmodell gezogen wird. Der Unterdruck ist nicht definiert und kann sich von Fall zu Fall stark unterscheiden. Es werden nasse und trockene Gipspositive als Tiefziehmodell eingesetzt, aber auch Schaumkörper. Oftmals wird Talkum als Gleitmittel verwendet. Diese Vorgehensweise zur Erstellung eines thermoplastischen Schaftes ist oft nicht erfolgreich, da die Entnahme der Platte aus dem Ofen zum exakt richtigen Zeitpunkt zu erfolgen hat. Anderenfalls wird der Plattendurchhang so groß, dass das Material zu stark ausdünnt und somit nicht mehr über die zur Verarbeitung notwendige Schmelzestabilität verfügt, beziehungsweise in Kontakt mit dem Boden des Ofens kommt.

Ein weiterer Nachteil dieses Verfahrens besteht darin, dass das Material amorph ist und durch das starke Verstrecken in Längsrichtung eine Längsausrichtung der Molekülketten einsetzt. Es treten Verstreckungsverhältnisse von bis zu 1:8 auf. Infolge der starken Verstreckung und sich üblicherweise ergebenden lokal unterschiedlichen Abkühlraten (z. B. dünne Lage auf nassem Gips/dickere Lage auf weniger nassem Gips) ergeben sich insbesondere bei amorphen Materialien eingefrorene Spannungen. Je stärker die eingefrorenen Spannungen sind, umso leichter kann der Schaft unter Beanspruchung reißen, d. h., dass das Material durch dieses Tiefziehverfahren sehr schlagempfindlich werden kann. Kommt es zu einem Riss im Material, breitet sich dieser sehr schnell und weit in Verstreckungsrichtung aus. Aufgrund der glasartigen, amorphen Struktur kann es dabei zu sehr scharfkantigen Rissen kommen, die zu erheblichen Verletzungen führen können. Auch ergeben sich durch den manuellen Tiefziehvorgang häufig unterschiedliche Wandstärken, z. B. durch lokal unterschiedliche Tiefziehgeschwindigkeiten. Diese unterschiedlichen Wandstärken tragen ebenfalls zu dem genannten Problem der hohen Schlagempfindlichkeit bei.

Aus WO 2008/092617 A1 ist bekannt, einen Schaft unter Verwendung eines von Haus aus konisch vorgeformten Kunststoffbauteil herzustellen. Hierzu wird, gemäß dem eigentlichen Erfindungskern der WO 2008/092617 A1, unter Verwendung einer mehrteiligen Schaftform, die an den Amputationsstumpf angelegt wird und deren einzelne flächige Teile relativ zueinander verschiebbar sind und in der gewünschten optimalen Relativstellung relativ zueinander wieder fixiert werden können, der Stumpf abgeformt. Sodann wird der Kunststoffrohling in die zu Stande gekommene Negativform eingesetzt und unter Verwendung heißer Luft, die in das Innere des Rohlings geblasen wird, erwärmt. Nach hinreichender Erwärmung wird durch einströmende Druckluft der thermoplastische Prothesenschaftrohling aufgeblasen und in Anlage an die Wandung der zuvor gebildeten Negativform gebracht. Nachteilig ist hierbei, dass durch Einblasen von Heißluft nur eine langsame Erwärmung des Prothesenschaftrohlings erreicht werden kann. Auch lässt sich keine gleichmäßige, homogene Durchwärmung erzielen, sondern nur ein von der Innenfläche des Rohlings ausgehendes Erwärmen. Findet sich an der Innenfläche des Rohling eine Stelle, deren Temperatur nennenswert über dem Durchschnitt liegt, wird dort die Druckluft das thermoplastische Material stärker verformen, da mit steigender Temperatur die Schmelzefestigkeit abnimmt. Dadurch dünnt sich das Material an dieser Stelle überdurchschnittlich aus, wodurch es sich noch stärker erhitzt, was wiederum zu einem verstärkten Ausbeulen führt, so dass der Rohling an dieser Stelle letztendlich platzen kann.

Der Erfindung liegt damit das Problem zugrunde, eine Erwärmungsvorrichtung anzugeben, die eine definierte, schnelle und homogene Er- bzw. Durchwärmung des Prothesenschaftrohlings ermöglicht.

Zur Lösung dieses Problems ist eine Erwärmungsvorrichtung vorgesehen, umfassend eine, vorzugsweise an einem Gestell angeordnete, Halterung für den Prothesenschaftrohling, sowie eine Heizeinrichtung in Form wenigstens eines Strahlungsheizers zum Erwärmen des Prothesenschaftrohlings, wobei die Halterung einen Teller zur Aufnahme des Prothesenschaftrohlings aufweist. Je nach Ausführung der Erfindung kann dieser Teller auch drehbar gelagert sein, damit der Prothesenschaftrohling während des Erwärmungsvorgangs relativ zum Strahlungsheizer über einen Motor bewegbar ist.

Die erfindungsgemäße Erwärmungsvorrichtung sieht den Einsatz wenigstens eines Strahlungsheizers zum Erwärmen des Prothesenschaftrohlings vor. Das Strahlungsspektrum des Strahlungsheizers ist so zu wählen, dass es gut genug vom thermoplastischen Material des Prothesenschaftrohlings absorbiert wird, um kurze Aufheizzeiten zu gewährleisten. Andererseits muss die Strahlung den Thermoplasten noch so gut durchdringen können, dass ein annähernd homogenes Durchwärmen des Prothesenschaftrohlings über die gesamte Wandstärke gewährleistet bleibt.

Als Strahlungsheizer wird bevorzugt ein Infrarotstrahler verwendet. Als Kunststoffmaterial wird besonders bevorzugt ein Copolyester, beispielsweise auf Terephthalsäurebasis, wie beispielsweise PET (Polyethylenterephthalat) verwendet. Verglichen mit den Erwärmungsvorrichtungen, wie sie im Stand der Technik bekannt sind (Umluftofen, Heißluft) ist beim erfindungsgemäßen Behandlungsgerät keine Vorwärmzeit erforderlich, insbesondere stellt sich eine sehr kurze Erwärmungszeit von üblicherweise maximal 5 Minuten ein, was mit den bekannten Heizmethoden nicht erreicht werden kann.

Weiterhin sind erfindungsgemäß zwei Methoden vorgesehen, um beim Aufheizen des Schaft-Rohlings zu gewährleisten, dass die Bereiche des Rohlings, die bestimmungsgemäß an die Stumpfform anzupassen sind, auf die angestrebte Verarbeitungstemperatur gebracht werden, ohne dabei Teilbereiche zu stark oder zu schwach zu erwärmen.

Die erste Methode besteht darin, dass der Prothesenschaftrohling auf einem drehbar gelagerten Teller angeordnet ist, wobei dieser Teller Teil einer Halterung ist, über die der Rohling im bzw. am Vorrichtungsgestell drehgelagert aufgenommen werden kann. Diese Drehlagerung ist dahingehend von Vorteil, als der Prothesenschaftrohling hierdurch über einen Motor gedreht werden kann. D. h., dass der Prothesenschaftrohling sich relativ zum lagefesten Strahlungsheizer dreht, an diesem also vorbeibewegt wird. Ist der Strahlungsheizer hierbei stabförmig ausgebildet, kann eine sehr homogene Erwärmung über die gesamte Länge des Rohlings erzielt werden, indem man den Abstand zwischen Strahler und Rohling passend justiert. Zu diesem Zweck können für verschiedene Größen von Rohlingen jeweils andere Fixierungspunkte zur Aufnahme der Strahler am Gerät vorgesehen werden. So lässt sich am komplett durchwärmten Rohling an allen Stellen die gleiche Weichheit erzielen, wodurch er optimal angeformt werden kann. Bei dieser Ausführung der Erfindung kann der Strahlungsheizer selbst außerhalb oder innerhalb des Prothesenschaftrohlings angeordnet sein, wobei die hierbei besonders bevorzugte Anordnung außerhalb des Prothesenschaftrohlings einen einfacheren Aufbau der Erwärmungsvorrichtung ermöglicht. Des Weiteren können stabförmige Strahler eingesetzt werden, die eine über ihre Länge gleich bleibende Strahlungsleistung aufweisen, was es möglich macht, kostengünstige, standardisierte Strahler einzusetzen. Grundsätzlich macht es für die Homogenität und Geschwindigkeit der Durchwärmung des thermoplastischen Materials keinen Unterschied, ob der oder die Strahlungsheizer außerhalb oder innerhalb des Rohlings angeordnet sind.

Die zweite Methode besteht darin, den oder die Strahlungsheizer auf der oder um die Symmetrieachse des Rohlings anzuordnen und die Strahlungsleistung des Strahlers oder der Strahler längs der Achse entsprechend dem Umfang des Rohlings zu variieren. Bei dieser Ausführung der Erfindung kann die Erwärmungsvorrichtung dank des Verzichts auf drehbar gelagerte Elemente einfach und kostengünstig gehalten werden. Neben stabförmigen Strahlern bietet sich alternativ bei dieser Ausführungsform an, angenähert punktförmige Stahler einzusetzen. Eine gleichmäßige Strahlungsintensität auf der Innenfläche der zu erwärmenden Stellen des Rohlings lässt sich hier erreichen, indem man den Abstand der angenähert punktförmigen Strahler zueinander in dem Maß reduziert, in dem eine Zunahme der Strahlungsleistung längs der Symmetrieachse gewünscht ist und/oder indem man die einzelnen Strahler in ihrer Strahlungsleistung regelt. Dabei kommt die auf einem Oberflächeninkrement des Rohlings auftreffende Strahlungsleistung durch Addition der von den einzelnen Strahlern auf dieser Fläche einfallenden Leistungen zustande.

Am den Prothesenschaftrohling aufnehmenden Teller ist ein Anschluss für eine Druckluft-Leitung zum Aufblasen des erwärmten Prothesenschaftrohlings oder zum Aufblasen eines am Teller angeordneten, den Prothesenschaftrohling innen auskleidenden, ballonartigen Aufblaselements vorgesehen. D. h., dass der Teller, der den Prothesenschaftrohling haltert und über den die Drehlagerung am Gestell möglich ist, darüber hinaus auch das Aufblasen des Rohlings ermöglicht. Er verbleibt also nach dem Erwärmen am Rohling, auch dann, wenn dieser aufgeblasen und an die Stumpfform angepasst wird. Somit wird nach ausreichendem Erwärmen der Prothesenschaftrohling mit dem Teller der Erwärmungsvorrichtung entnommen und beispielsweise in die den Amputationsstumpf negativ abbildende Form gesetzt. Durch Einblasen von Druckluft wird der Rohling aufgeblasen, so dass er sich optimal an die Form anlegt. Zwischen Teller und Prothesenschaftrohling kann ein entsprechendes Dichtmittel eingebracht werden, um eine dichte Verbindung zwischen Teller und dem Rand des Rohlings zu ermöglichen. Alternativ kann ein ballonartiges Aufblaselement vorgesehen sein, das am Teller angeordnet ist und über das beispielsweise direkt die Abdichtung zum Prothesenschaftrohling erfolgt. Wird das Aufblaselement aufgeblasen, so dehnt sich dieses aus und legt sich an die Innenwand des Rohlings an. Bei weiterem Aufblasen wird hierüber auch zwangsläufig der Rohling aufgeweitet, so dass er sich von innen flächig an die Negativform anlegt. Wird bei innenliegendem Strahler ein Aufblaselement verbaut, ist es von Vorteil, das Aufblaselement so auszuführen, dass es von der erwärmenden Strahlung möglichst gut durchdrungen werden kann.

Wie bereits beschrieben, wird bei der Ausführung mit drehbar gelagertem Teller besonders die Ausführungsform mit einem oder mehreren außerhalb des Rohlings angeordneten Strahlungsheizern bevorzugt. Bei dieser ist eine zentrisch am Teller befestigte, vorzugsweise teleskopierbare Stange vorgesehen, die den Prothesenschaftrohling axial durchdringt und am geschlossenen Ende des Prothesenschaftrohlings durch ein sich an dieser Stelle befindliches Loch wieder austritt. Somit wird der Rohling um seine Symmetrieachse drehbar am Gestell aufgenommen. Diese Stelle dient als zweiter Lagerpunkt der Drehlagerung des Rohlings. Die Stange ist also ebenfalls Teil der Halterung. Der Rohling ist somit einerseits über den Teller respektive ein am Teller vorgesehenes Lagerelement, andererseits über die Stange drehgelagert. Die Stange kann auch das zuvor beschriebene, ballonartige Aufblaselement durchdringen, welches somit die Stange umgibt. Sie ist auch in dieser Ausführungsform bevorzugt teleskopierbar, wodurch ermöglicht wird, den Prothesenschaftrohling zwecks Anpassung an die Außenform in diese durch Verlängern der Stange hineinzudrücken.

Eine Alternative zu dem beschriebenen Blasformprozess, bei dem der erwärmte Rohling in eine hohle Stumpfform eingeblasen wird, ist das Anformen des erhitzten Prothesenschaftrohlings an eine den Stumpf abbildende Positivform. Um dies zu ermöglichen, ist eine alternative Ausführung der Erwärmungsvorrichtung vorgesehen. Bei dieser ist der Strahlungsheizer ebenfalls außerhalb des Prothesenschaftrohlings angeordnet, am Teller befindet sich jedoch ein Stützdom, der im Wesentlichen der Innenform des Prothesenschaftrohlings entspricht, so dass der Prothesenschaft Rohling auf diesen aufgesetzt werden kann. Der Stützdom ist vorzugsweise luftdurchlässig, rotationssymmetrisch und seine Rotationsachse entspricht der des Drehtellers. Bei dieser Erfindungsausgestaltung wird also auf den drehgelagerten Teller zunächst ein Stützdom aufgesetzt, auf den wiederum der Prothesenschaftrohling gesetzt wird, der, über geeignete Verbindungsmittel abgedichtet, ebenfalls mit dem Teller gekoppelt wird. Somit wird der Prothesenschaftrohling während der Erwärmung gegen Einsinken oder Verformung abgestützt, da er vollflächig auf dem Stützdom aufliegt. Nach dem Durchwärmen kann er, analog einer durchwärmten Tiefziehplatte, zu einem Prothesenschaft weiterverarbeitet, also z.B. über ein Gipspositiv gezogen werden.

Dieser Stützdom kann vorteilhafterweise auch so ausgestaltet werden, dass seine Oberfläche die Heizstrahlung reflektiert, wodurch sich der Anteil der Strahlung, der zur Erwärmung des Rohlings genutzt wird, erhöht. Dieses Prinzip der Effizienzsteigerung der Erfindung lässt sich auch auf Ausführungen mit im Inneren des Rohlings angeordneten Heizstrahlern übertragen. Dazu ist ein den Rohling umfangender Reflektor so auszubilden, dass der Rohling, mit dem geschlossenen Ende nach unten vollflächig mit seinen zu erwärmenden Bereichen auf dem Reflektor aufliegt, wodurch, nach dem Erweichen des Rohlings, auch einer Verformung durch sein Eigengewicht entgegengewirkt wird.

Eine weitere alternative Ausführung mit einem oder mehreren außen liegenden Strahlungsheizern besteht darin, auf dem Drehteller ein Positivmodell des Stumpfes zu befestigen und darüber, zum Drehteller hin abgedichtet, den Rohling zu befestigen. Nach hinreichender Erwärmung wird durch Anlegen von Unterdruck zwischen Rohling und Positiv dann der Prothesenschaftrohling an das Positiv gesaugt. Dazu ist das Positiv optional mit einer dünnen luftdurchlässigen Schicht, wie etwa einem dünnen Strumpf, zu überziehen.

Bei den beiden letztgenannten Ausführungen kann entweder auf die das Positiv und den Rohling durchdringende Stange verzichtet werden, was eine drehbare Lagerung dieser Anordnung allein über den Teller erfordert, oder es wird wiederum ein zweiter Lagerungspunkt über einen den Prothesenschaftrohling am geschlossenen Ende durchdringenden Stift realisiert, der in eine entsprechende Aufnahme am Gestell einrastet.

Hinsichtlich der Drehlagerung des Prothesenschaftrohlings sind unterschiedliche Ausgestaltungen denkbar. Entweder kann der Teller oder die Stange mit dem Motor tellerseitig gekoppelt sein, d. h. dass der Motor seine Antriebsleistung an die Halterung, dort also an den Teller oder an die den Prothesenschaftrohling durchdringende Stange überträgt. Denkbar ist es aber auch, dass das geschlossene Ende des Prothesenschaftrohlings in einer drehbar gelagerten Aufnahme fixiert ist, die mit dem Motor gekoppelt ist, während sich der Teller frei dreht.

In Weiterbildung der Erfindung kann das Gestell einen in seiner Höhe verstellbaren Abschnitt aufweisen, durch den sich unterschiedliche Abstände zwischen Teller und der Aufnahme für das geschlossene Ende des Prothesenschaftrohlings einstellen lassen. Dies ermöglicht es, das Gestell an die Größe des Prothesenschaft-Rohlings anzupassen, da die Herstellung unterschiedlich großer Prothesenschäfte, die ja den vorhandenen anatomischen Gegebenheiten entsprechen müssen, unterschiedlich große Schaft-Rohlinge erfordern kann.

Wenngleich es grundsätzlich ausreichend ist, bei der Ausführung mit außenliegendem Strahler nur einen Strahlungsheizer zu verwenden, sieht eine zweckmäßige Weiterbildung den Einsatz zweier oder mehrerer um den Umfang des Prothesenschaftrohlings verteilt angeordneter Strahlungsheizer vor.

Insbesondere bei außen liegender Anordnung des oder der Strahlungsheizer können zweckmäßigerweise ein oder mehrere Abschirmungen, die gegebenenfalls auch reflektierend für die emittierte Strahlung sind, vorgesehen sein. Dies bietet den Vorteil, dass weniger unnötige Erwärmung der Vorrichtungsumgebung respektive der Vorrichtung selbst stattfindet. Eine reflektierende Ausbildung der Abschirmungen ist zweckmäßig, da bei geeigneter Ausgestaltung hierüber die in Richtung der Abschirmung abgegebene Wärmestrahlung zum Rohling hin reflektiert werden kann, um auch diesen Strahlungsanteil zur Erwärmung zu nutzen. Durch entsprechende Ausgestaltung der Abschirmbleche kann auch eine Strahlungskonzentration erfolgen, die den Abstrahlwinkel so einengt, dass die Wärmestrahlung gezielt auf den Rohling appliziert werden kann. Die Abschirmung kann auch z.B. in Form einer nicht umfänglichen Verspiegelung in den Strahler selbst integriert werden. In diesem Fall ist apparativ noch ein Schutz vor Verbrennungen infolge unbeabsichtigter Berührung des Strahlers in die Apparatur zu integrieren.

Eine weitere, besonders zweckmäßige Ausführung der Erfindung sieht vor, eine Steuerungseinrichtung einzusetzen, der ein Temperatursensor zum Erfassen der Temperatur des Prothesenschaftrohlings zugeordnet ist, wobei die Steuerungseinrichtung den Betrieb des oder der Strahlungsheizer in Abhängigkeit des Sensorerfassungsergebnisses steuert. Dies bietet die Möglichkeit, einen von der Temperatur des Prothesenschaftrohlings abhängigen Betrieb zu realisieren Als Sensor kann beispielsweise ein Infrarotthermometer verwendet werden. Somit kann beispielsweise der Strahlungsheizer solange in Betrieb sein, bis eine definierte Grenztemperatur erreicht ist, woraufhin der Strahlungsheizer abgestellt wird. Auch kann in der Steuerungseinrichtung ein oberer und einen unterer Temperaturschwellenwert hinterlegt werden, wobei die Steuerungseinrichtung den Strahlungsheizer bei Erfassen einer dem oberen Temperaturschwellwert entsprechenden Temperatur ausschaltet und nach Abkühlen bei Erfassung einer dem unteren Temperaturschwellwert entsprechenden Temperatur wieder einschaltet. D. h. dass ein Temperaturfenster definiert ist, in dem der Erwärmungsprozess und darauf gestützt, nachfolgend auch der Umformprozess erfolgen soll. Wird beim Aufheizen eine Temperatur des Rohlings erfasst, die dem oberen Temperaturschwellwert entspricht, so ist der Rohling prozessfähig, kann also verformt werden. Optional wird dann ein Signal ausgegeben und der Strahlungsheizer wird entweder sofort oder mit Verzögerung abgeschaltet. Er kann sich aber beispielsweise noch weiterdrehen. Wird der Rohling nicht sofort entnommen und weiterverarbeitet, so sinkt die Oberflächentemperatur des Rohlings wieder. Wird der untere Temperaturschwellwert erreicht, so wird der Strahlungsheizer wieder eingeschaltet, um den Rohling im Temperaturfenster zu halten. Dieser Warmhalte- oder Hysteresemodus ist optional zeitlich zu begrenzen, um etwa einer thermischen Schädigung des Thermoplasten vorzubeugen. Anschließend wird beispielsweise ein weiteres Signal ausgegeben, das anzeigt, dass sich die Erwärmungsvorrichtung nun komplett abschaltet und keine weitere Temperaturkontrolle respektive Erwärmung des Rohlings mehr erfolgt. Ebenso ist eine Leistungsregelung des oder der Strahler zur Temperaturkontrolle denkbar.

Besonders bevorzugt ist die Ausführung, bei der angenähert punktförmige, längs um die Symmetrieachse im Inneren des Rohling angeordnete Strahler leistungsgeregelt betrieben werden müssen, da bei vorhandener, oben beschriebener Temperaturregelung die Leistungsregelung dieser Strahler keine zusätzliche Hardware erfordert und somit keine zusätzlichen Produktionskosten entstehen.

Weitere Vorteile, Merkmale und Einzelheiten der Erfindung ergeben sich aus den im Folgenden beschriebenen Ausführungsbeispielen sowie anhand der Zeichnungen. Dabei zeigen:
- Fig. 1: eine Prinzipdarstellung einer erfindungsgemäßen Erwärmungsvorrichtung in einer ersten Ausführungsform,
- Fig. 2: eine Prinzipdarstellung einer erfindungsgemäßen Erwärmungsvorrichtung in einer zweiten Ausführungsform,
- Fig. 3: eine Prinzipdarstellung einer erfindungsgemäßen Erwärmungsvorrichtung in einer dritten Ausführungsform,
- Fig. 4: eine Prinzipdarstellung einer erfindungsgemäßen Erwärmungsvorrichtung in einer vierten Ausführungsform,
- Fig. 5: eine Prinzipdarstellung einer erfindungsgemäßen Erwärmungsvorrichtung in einer fünften Ausführungsform
- Fig. 6: eine Prinzipdarstellung einer erfindungsgemäßen Erwärmungsvorrichtung in einer sechsten Ausführungsform mit den Teildarstellungen Fig. 6a und Fig. 6b,
- Fig. 7: eine Prinzipdarstellung einer erfindungsgemäßen Erwärmungsvorrichtung in einer siebten Ausführungsform,
- Fig. 8: eine Seitenansicht einer mehrere einzelne Strahlungsheizer aufweisenden Strahlereinheit, und
- Fig. 9: eine Aufsicht auf die Strahlereinheit aus Fig. 8.

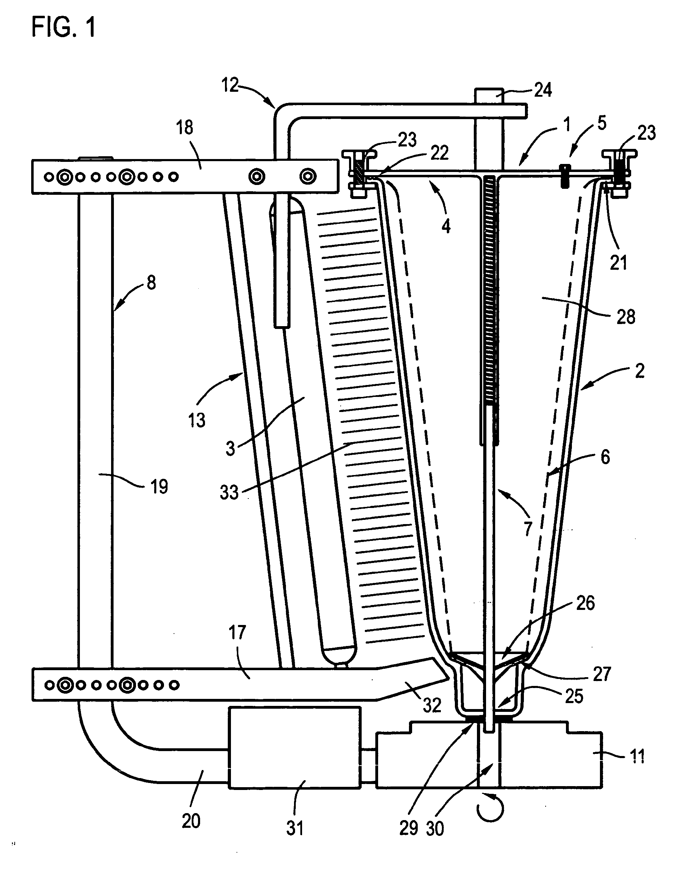

Fig. 1 zeigt eine erfindungsgemäße Erwärmungsvorrichtung, umfassend ein Gestell 8, an dem eine Heizeinrichtung in Form eines Strahlungsheizers 3 vorgesehen ist. Der Strahlungsheizer ist an einem unteren Träger 17 und einem oberen Träger 18 angeordnet, wobei zumindest der obere Träger, bevorzugt aber auch der untere Träger an einem Vertikalträger 19 des Gestells 8 horizontal verschiebbar und/oder verschwenkbar angeordnet sind. Dies ermöglicht es, die Position und räumliche Ausrichtung des Strahlungsheizers 3 bezüglich der Drehachse des Prothesenschaftrohlings 2 verändern zu können, um die Apparatur an verschiedene Größen von Rohlingen anzupassen.

Dem Strahlungsheizer 3, beispielsweise einem Infrarotstrahler, ist eine Abschirmung 13 zugeordnet, die die emittierte Strahlung zur Umgebung hin abschirmt, respektive zum Strahlungsheizer 3 und zum Prothesenschaftrohling zurückreflektiert.

Vorgesehen ist ferner ein unterer horizontaler Träger 20 als Teil des Gestells 8, an dem ein Motor 11 angeordnet ist, der zum drehenden Antreiben eines nachfolgend noch beschriebenen Prothesenschaftrohlings 2 aus Kunststoff dient. Am oberen Träger 18 ist ein Halter 12 angeordnet, der schwenkbar oder horizontal verschiebbar sowie in seiner Höhe verstellbar ist. Dieser dient als Aufnahme respektive Drehlagerung für eine nachfolgend noch beschriebene Halterung, mit der der bereits genannte Prothesenschaftrohling drehgelagert ist. Über den Strahlungsheizer 3 ist wie beschrieben der Prothesenschaftrohling 2, der beispielsweise aus transparentem Kunststoff, beispielsweise einem amorphen, aromatischen Copolyester besteht, zu erwärmen.

Um den Prothesenschaftrohling 2 gestellseitig positionieren und aufnehmen zu können, ist eine Halterung vorgesehen, umfassend einen Teller 4, auf den der Prothesenschaftrohling mit seinem flanschartigen Rand 21 aufgesetzt ist. Zwischen Teller 4 und Rand 21 ist ein Dichtring 22 angeordnet. Über geeignete Befestigungsmittel 23, z. B. Schrauben oder Klammern, erfolgt so eine druckdichte Anbindung des Prothesenschaftrohlings 2 an den Teller 4. Am Teller 4 ist ein Lagerzapfen 24 vorgesehen, der eine entsprechende Drehlagerdurchbrechung am Halter 12 durchsetzt. Hierüber wird die obere Drehlagerung des Prothesenschaftrohlings 2 realisiert. Gezeigt ist ferner ein Anschluss 5, an den ein Druckluftschlauch angeschlossen werden kann, der dazu dient, den erwärmten Prothesenschaftrohling 2 nach seiner Entnahme aus der Erwärmungsvorrichtung und Einsetzen in eine von ihm auszufüllende Form aufzublasen.

Vorgesehen ist ferner eine teleskopierbare Stange 7, die am Teller 4 angeordnet ist und die durch einen Durchbruch 25 am unteren Ende des

Prothesenschaftrohlings 2 geführt ist. Die teleskopierbare Stange 7 ist mit einer im Wesentlichen leicht konischen Scheibe 26 verbunden, die ein Dichtelement 27 gegen die an dieser Stelle trichterförmige Innenfläche des Prothesenschaftrohlings 2 drückt, so dass in diesem Bereich eine umfängliche radiale Abdichtung des Rohling-Innenraums 28 gegeben ist. Über eine Mutter 29, die auf ein sich in diesem Bereich befindendes Außengewinde der Stange 7 aufgeschraubt wird, wird sichergestellt, dass der Prothesenschaftrohling ausreichend gegen das Dichtelement 27 gedrückt wird, um Dichtigkeit zu gewährleisten. Des weiteren greift die teleskopierbare Stange 7 formschlüssig in eine hier nur dem Prinzip nach dargestellte Abtriebswelle 30 des Motors 11 ein, hierüber erfolgt also die Einkopplung des Antriebs. Die Stange ist hierzu im unteren Bereich bevorzugt viereckig ausgebildet. D. h., dass hierüber bei Betrieb des Motors 11 der gesamte Prothesenschaftrohling 2 gedreht werden kann.

Über eine Steuerungseinrichtung 31, die hier nur dem Prinzip nach dargestellt ist, wird die Erwärmungsvorrichtung gesteuert. Soll ein zuvor an der Halterung 1 umfassend Teller 4 und Stange 7 etc. fixierter Prothesenschaftrohling 2 erwärmt werden, so wird er zunächst gestellseitig aufgenommen. Anschließend wird durch entsprechende Verstellung über die Träger 17, 18 der Strahlungsheizer 3 positioniert, was mitunter erforderlich ist, da unterschiedlich große Prothesenschaftrohlinge 2 eingesetzt werden können. Anschließend steuert die Steuerungseinrichtung 31 den Motor 11 sowie den Strahlungsheizer 3 an. Der Prothesenschaftrohling 2 dreht also am Strahlungsheizer 3 vorbei, der Strahlung 32, also beispielsweise Infrarotstrahlung, zum Prothesenschaftrohling 2 hin emittiert. Das Strahlungsspektrum ist so gewählt, dass es optimal mit dem Kunststoffmaterial des Prothesenschaftrohlings 2 wechselwirkt, dass es also einerseits ausreichend gut in das Kunststoffmaterial eindringt und andererseits auch gut absorbiert wird, so dass es zu einer möglichst gleichmäßigen, homogenen Durchwärmung über die gesamte Materialstärke bei möglichst hohem Wirkungsgrad kommt. Über die Steuerungseinrichtung 31 kann optional auch die Strahlungsintensität eingestellt werden. Über die Abschirmung 13, sowie den Abschirmfortsatz 32 am unteren Horizontalträger 17 wird sichergestellt, dass die emittierte Wärmestrahlung 32 primär auf die zu erwärmenden Bereiche des Prothesenschaftrohlings 2 trifft.

In der Steuerungseinrichtung 31 sind bevorzugt zwei Temperaturschwellwerte abgelegt, ein oberer und ein unterer. Über einen geeigneten Temperatursensor, beispielsweise einen Infrarotsensor, ist es nun möglich, die Oberflächentemperatur des Prothesenschaftrohlings 2 zu messen. Die Steuerung erfolgt nun derart, dass bei Erfassen einer Oberflächentemperatur, die dem oberen Schwellwert entspricht, die Steuerungseinrichtung 31, die mit dem Temperatursensor kommuniziert, den Betrieb des Strahlungsheizers 3 abstellt, während der Motor 11 nach wie vor dreht. Es kommt zwangsläufig zu einer langsamen Abkühlung. Mit Erreichen des unteren Temperaturschwellwerts steuert die Steuerungseinrichtung 31 den Strahlungsheizer 3 wieder an, d. h., dass der Prothesenschaftrohling 2 wieder erwärmt wird. Es ist also ein Temperaturfenster definiert, innerhalb dem sich die Temperatur des Rohlings bewegt. Diese Hysterese kann mehrfach durchlaufen werden. Wird nach erstmaligem Erreichen der Zieltemperatur der Prothesenschaftrohling 2 nicht entnommen, um weiterverarbeitet zu werden, kann ein Zeitintervall hinterlegt werden, während dem der Rohling auf verarbeitungsfähiger Temperatur gehalten wird. Nach Durchlaufen dieses Zeitintervalls erfolgt eine endgültige Abschaltung, um eine thermische Beeinträchtigung des thermoplastischen Materials zu vermeiden. Bei jedem Erreichen des oberen Temperaturwerts wird beispielsweise über ein geeignetes Signal (akustisch oder optisch) dem Anwender mitgeteilt, dass der Prothesenschaftrohling 2 verarbeitungsfähig ist. Ein anderes Signal wird ausgegeben, wenn der Heizbetrieb vollständig eingestellt wird. Wie bereits beschrieben erfolgt das Anpassen des erwärmten Prothesenschaftrohlings 2 an die hier nicht näher gezeigte Stumpfform bei einer Apparatur nach Fig. 1 dadurch, dass Druckluft über den Anschluss 5 eingedrückt wird um den Rohling 2 zu verformen Bei dieser Ausgestaltung beaufschlagt die Druckluft unmittelbar die Innenfläche des Rohlings. Gestrichelt dargestellt ist jedoch ein in seinem Inneren angeordnetes, ballonartiges Aufblaselement 6, das an Stelle des Dichtrings 22 im oberen Bereich zwischen Teller 4 und dem Rand des Rohlings 21 verspannt wird und dergestalt die Abdichtung realisiert. Am unteren Ende kann das Aufblaselement 6 beispielsweise am Dichtelement 27 befestigt sein. Wird nun Luft eingeblasen, so verformt sich das Aufblaselement 6 und legt sich an die Innenwand des Rohlings 2 an, bei weiterem Aufblasen wird der Rohling ebenfalls aufgeweitet. Da die Erfindung das Aufblaselement 6 nur optional beinhaltet, ist es gestrichelt dargestellt.

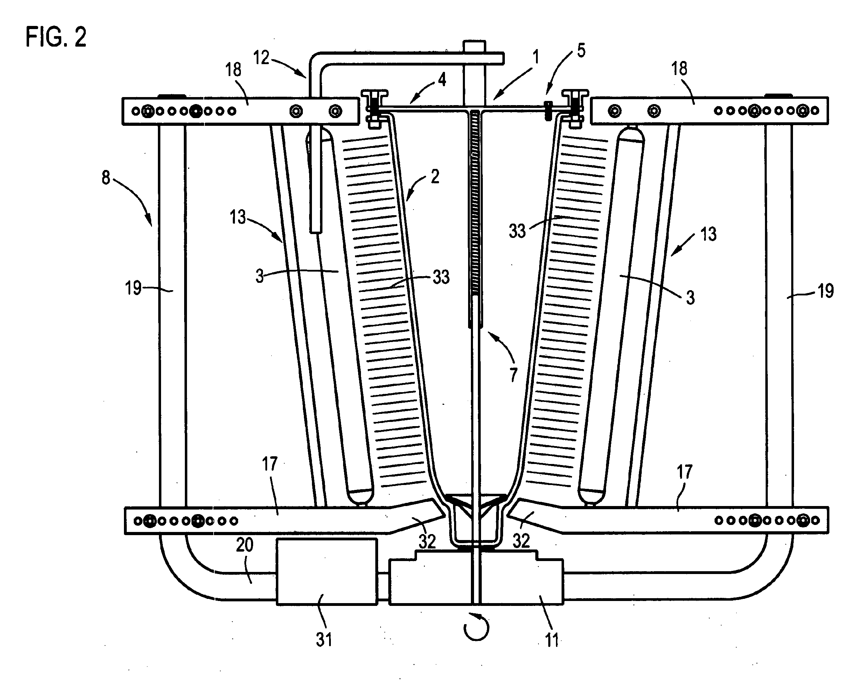

Fig. 2 zeigt eine erfindungsgemäße Ausgestaltung der Erwärmungsvorrichtung, bei der zwei um 180° versetzt angeordnete Strahlungsheizer 3 vorgesehen sind. D. h., dass das Gestell 8 letztlich zwei erste und zweite Träger 17 und 18, sowie zwei Vertikalträger 19 und einen gemeinsamen unteren Träger 20 aufweist. Wiederum ist jedem Strahlungsheizer 3 wenigstens eine Abschirmung 13 zugeordnet. Die Steuerungseinrichtung 31 steuert den Betrieb beider Strahlungsheizer 3, die bevorzugt synchron betrieben werden. Durch Verwendung zweier Strahlungsheizer 3 erfolgt ein simultaner Energieeintrag an zwei Stellen, was für eine gleichmäßigere und schnellere Durchwärmung von Vorteil ist. Ansonsten wird diese Ausgestaltung der Erfindung entsprechend der aus Fig. 1 bekannten Vorrichtung betrieben.

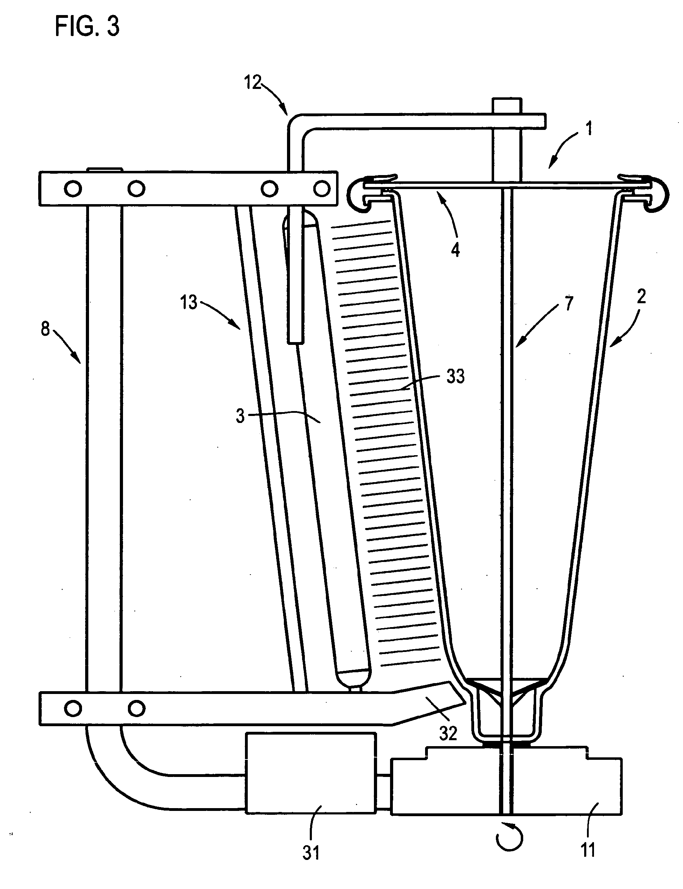

Fig. 3 zeigt eine weitere Ausführungsform einer erfindungsgemäßen Erwärmungsvorrichtung, bei der das Gestell 8 dem Gestell 8 aus Fig. 1 entspricht. Anders als bei den bisherigen Ausführungsformen wird nachfolgend der Prothesenschaftrohling 2 nicht blasgeformt, sondern tiefgezogen. Zu diesem Zweck ist es nicht erforderlich, den Rohling 2 luftdicht zu verschließen. Aus diesem Grund ist der Teller 4 auch nur so ausgeführt, dass er über einfache Schnellspannelemente 34 mit dem Rand des Rohlings 21 zu verbinden ist. Ein Druckluftanschluss ist hier nicht erforderlich. Die Stange 7 ist hier nicht teleskopierbar, denn sie verbleibt während des nachfolgenden Verformungsvorgangs des Rohling 2 nicht im Rohling (anders bei den Ausführungen nach den Fig. 1 und 2). Vielmehr wird nach hinreichender Erwärmung des Prothesenschaftrohlings 2 der Teller 4 samt der an ihm befindlichen Stange 7 nach Öffnen der Schnellspannelemente 34 dem Rohling entnommen. Dieser wird sodann in eine Tiefziehvorrichtung überführt, wo er durch Anlegen von Unterdruck an die Stumpfform angepasst wird.

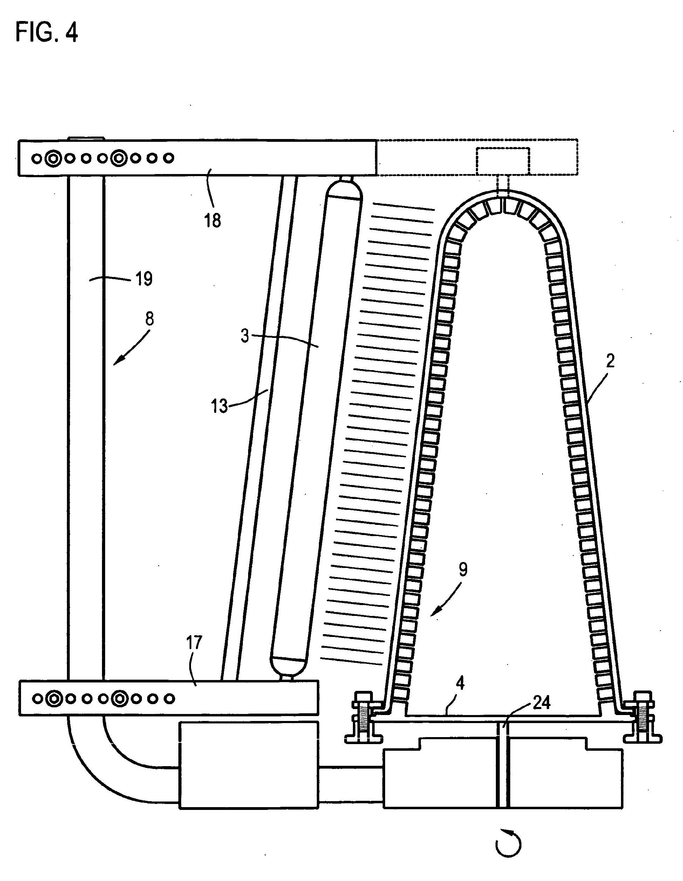

Fig. 4 zeigt eine vierte Ausführungsform einer Erwärmungsvorrichtung, die wiederum ein Gestell 8 aufweist, das von seiner Funktionalität her dem Gestell aus Fig. 1 entspricht. Es ist wiederum ein unterer Träger 20 nebst Motor 11, ein vertikaler Träger 19, sowie ein unterer Träger 17 und ein oberer Träger 18, wobei die beiden Träger 17 und 18 schwenkbar am Vertikalträger 19 angebracht sind. Vorgesehen ist wiederum ein Strahlungsheizer 3 nebst Abschirmung 13. Anders als bei den bisherigen Ausführungsformen wird hier jedoch der Prothesenschaftrohling 2 mit der Öffnung nach unten weisend eingebracht. Vorgesehen ist wiederum ein Teller 4 nebst Drehlagerungszapfen 24, wobei der Drehlagerungszapfen 24 direkt mit der nicht näher gezeigten Motorabtriebswelle gekoppelt wird, hierüber erfolgt also der Drehantrieb des Tellers 4. Am Teller 4 ist ein Stützdom 9 angeordnet. Dieser ist entweder luftdurchlässig oder so ausgeführt, dass er eine Vielzahl von Luftdurchlässen [...] aufweist. Wenn der erwärmte Schaft durch Tiefziehen weiterverarbeitet wird, muss der Stützdom jedoch nicht luftdurchlässig sein. Über diesen Stützdom 9 wird der Prothesenschaftrohling 2 gesetzt, der wiederum über entsprechende Befestigungsmittel 23 unter Verwendung eines Dichtrings 22 mit dem Teller 4 verbunden wird. Dieser Stützdom 9 verhindert ein Einsinken des Prothesenschaftrohlings 2, wenn dieser erwärmt ist. Er bewirkt also, dass dieser seine Form beibehält. Zu diesem Zweck hat der Stützdom möglichst exakt der Innengeometrie des Rohlings zu entsprechen und sollte aus einem Material gefertigt sein, dass beim Erhitzen möglichst wenig Wärme von der Innenseite des Rohlings abführt. Gestrichelt gezeigt ist ferner ein optional vom Stützdom 9 vertikal nach oben abstehender Stift 10, der, sofern vorgesehen, in eine entsprechende Drehlageraufnahme 36 am dann verlängert ausgebildeten oberen Träger 18 (verlängerter Bereich ebenfalls nur gestrichelt gezeigt) eingreift. Auf diese Weise kann eine obere Drehlagerung realisiert werden. Ausreichend ist bei dieser Ausgestaltung aber bereits die untere Drehlagerung über den Drehlagerzapfen 24. Im Betrieb wird der Prothesenschaftrohling 2 durch den Strahlungsheizer 3 erwärmt. Nach Erreichen der gewünschten Prozesstemperatur wird der Teller 4 samt Stützdom 9 und Prothesenschaftrohling 2 entnommen und zur Weiterbearbeitung z.B. durch Tiefziehen in die Schaftnegativform eingesetzt.

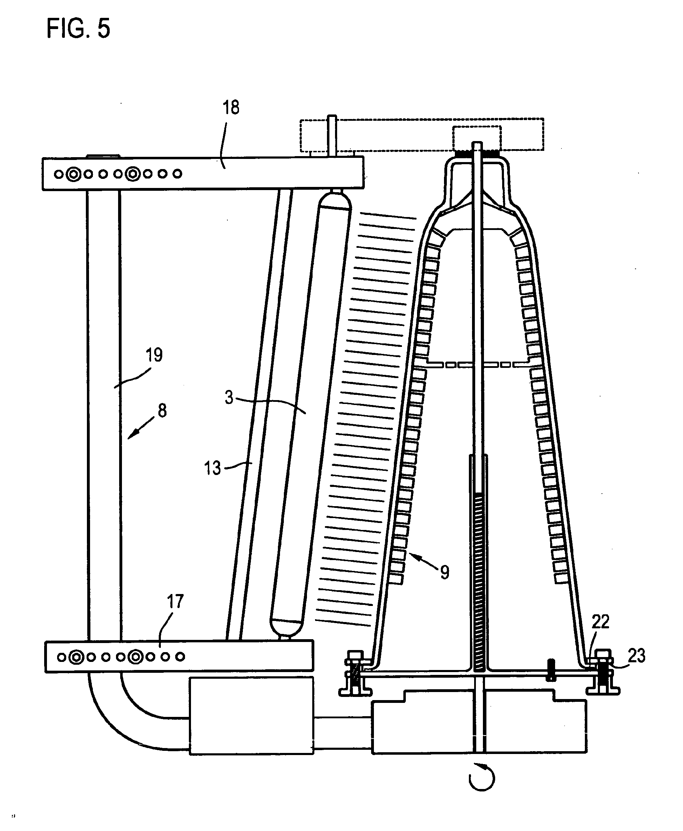

Fig. 5 zeigt eine im wesentlichen Fig. 4 entsprechende Ausführungsform, bei der jedoch eine teleskopierbare Stange 7 vorgesehen ist, die mit dem Motor 11 gekoppelt und oberseitig drehgelagert ist. Auch hier ist im Inneren des Prothesenschaftrohlings ein Stützdom 9 vorgesehen, der luftdurchlässig ist oder Luftdurchlässe aufweist. Nach dem Erwärmen wird der Rohling nebst Teller 4 und Stange 7 entnommen und in eine Schaftnegativform eingesetzt. Sodann wird über den hier vorgesehenen Druckluftanschluss 5 Luft eingeblasen, wodurch der Prothesenschaftrohling 2 durch den luftdurchlässigen inneren Stützdom 9 aufgeblasen wird.

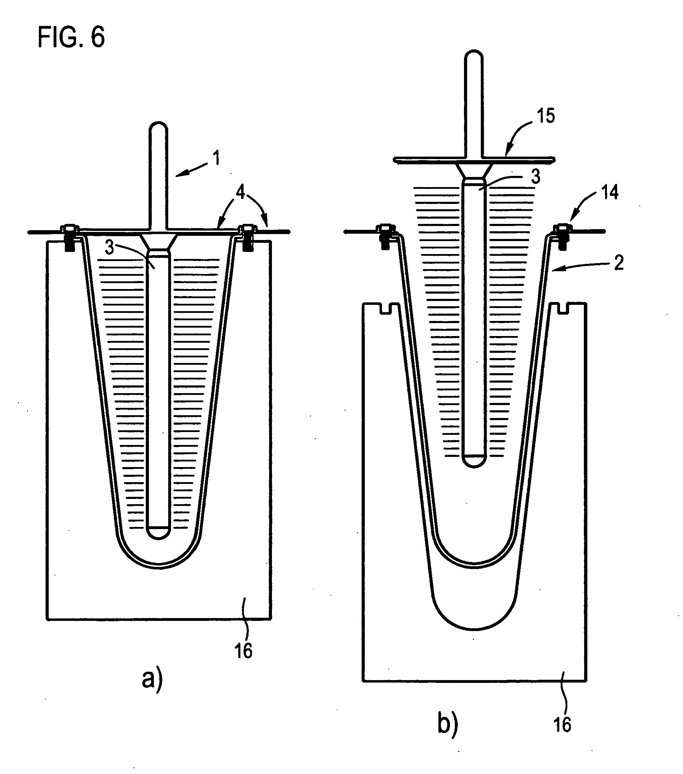

Fig. 6 mit den Teilfiguren 6a und 6b zeigt eine fünfte Ausführungsform einer Erwärmungsvorrichtung, bei der der Prothesenschaftrohling 2 durch einen stabförmig ausgebildeten Strahler 3 erwärmt wird, der sich während des Erwärmungsvorgangs auf der Symmetrieachse des Rohlings befindet, so dass umlaufend betrachtet der Abstand zwischen Strahler und Oberfläche des Rohlings gleich bleibt. Entlang des der Längsachse des Strahlers nimmt der Abstand zwischen der Oberfläche des Rohlings und der Oberfläche des Rohlings zum geschlossenen Ende des Rohlings hin ab. Um dennoch eine gleichmäßige Erwärmung der zu erwärmenden Bereiche des Rohlings zu gewährleisten, nimmt die Strahlungsleistung des Strahlers zum geschlossenen Ende des Rohlings hin so ab, dass dennoch auf jedem Flächeninkrement der zu erwärmenden Bereiche des Rohlings dieselbe Strahlungsleistung auftrifft. Aufgenommen wird der Rohling 2 bei dieser Ausführungsform der Erfindung durch einen idealer Weise die Heizstrahlung reflektierenden Isolationskörper 16. Dieser stützt den Rohling 2 vollflächig ab, sodass er sich auch im erweichten Zustand nicht deformiert. Der obere Rand des Rohlings ist mit dem Teller 4 verschraubt, der hier dreiteilig ausgebildet ist und auf dem Isolationskörper 16 aufliegt, vgl. Fig. 6a.

Um den Rohling 2 mittels Tiefziehverfahren weiterverarbeiten zu können, wird der Strahler zusammen mit dem Innenteller 15 des hier zweiteiligen, aus dem Innenteller 15 und dem Außenring 14 bestehenden Tellers 4 aus dem Rohling entnommen (siehe Fig. 6b), worauf sich der Rohling 2 aus dem isolierenden Stützkörper 16 mithilfe des Außenringes 14 des Tellers 2 entnehmen lässt. Am Außenring 14 ist der Prothesenschaftrohling 2 mithilfe von Befestigungsmitteln 23 und einem zweiten Ring 35 befestigt. Die weitere Verarbeitung erfolgt analog der Verarbeitung von Tiefziehplatten.

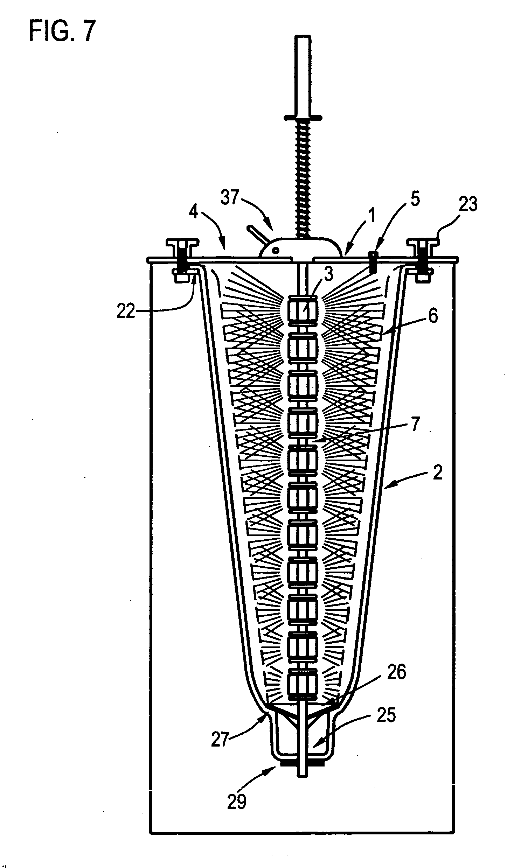

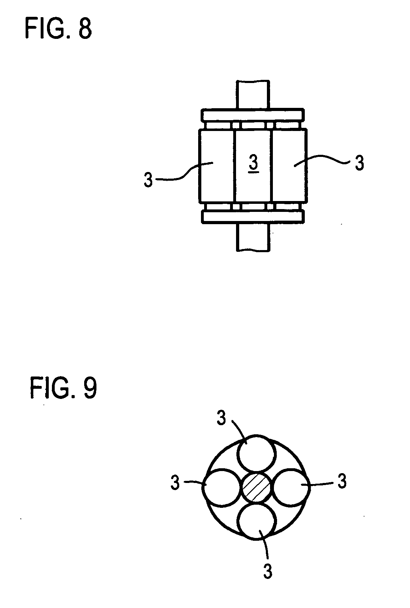

Die Fig. 7 - 9 zeigt eine sechste Ausführungsform einer Erwärmungsvorrichtung, die prinzipiell der Ausführungsform aus Fig. 6 entspricht. Anstatt eines stabförmig ausgebildeten Heizstrahlers weist Fig. 7 viele angenähert punktförmige Heizstrahler 3 auf, die um die Symmetrieachse des Prothesenschaftrohlings 2 herum angeordnet sind, siehe die Fig. 8 und 9, die eine einzelne Strahlereinheit in einer Seitenansicht und einer Aufsicht zeigen. Eine solche Strahlereinheit kann als ein eigenständiges Bauteil, das einzeln an der Stange 7, die es durchsetzt, angebaut werden kann, ausgeführt sein. Die einzelnen Heizstrahler 3 sind hinsichtlich ihrer Strahlungsleistung so ausgewählt bzw. leistungsgeregelt, das sich ihre Einzelstrahlungsfelder auf der Oberfläche des Prothesenschaftrohlings so überlagern, dass auf jedem Flächeninkrement der zu erwärmenden Bereiche des Rohlings dieselbe Strahlungsleistung auftrifft. Als Strahler können hier beispielweise Sofitten eingesetzt werden.

Fig. 7 zeigt eine Ausführungsform, die es erlaubt, den Prothesenschaftrohling 2 im oben beschriebenen Blasformverfahren zu verarbeiten. Zu diesem Zweck sind die in Fig.1 beschriebenen Elemente 4, 5, 6, 22, 23, 25, 26, 27 und 29 mit gleicher Funktionalität wie bei Fig. 1 auch Bestandteil dieser Ausführungsform.

Da es bei der Anfertigung von Prothesenschäften mitunter notwendig ist, das offene Ende trichterförmig aufzuweiten, um z.B. bei einem Oberschenkelschaft eine Aufsitzzone zu schaffen, ist es bei der Verarbeitung des Rohlings durch Blasformen sinnvoll, während des Blasformvorgangs die Länge des Rohlings verkürzen zu können. Bei der dargestellten Ausführungsform wird das erreicht, indem sich die Stange 7 nach entriegeln einer Klemmvorrichtung 37, durch die die Stange 7 abgedichtet durchgeführt wird, federunterstützt aus dem Teller 4 herausziehen lässt. In der dargestellten Ausführungsform beläuft sich der Weg, um den die Stange 7 herausgezogen werden kann, maximal auf die Distanz zwischen Teller 4 und der obersten Strahler-Einheit. Soll während der Verarbeitung des Rohlings mit einem Erwärmungsgerät dieser Bauart eine größere Längenverkürzung realisiert werden können, besteht entweder die Möglichkeit, die oberen Strahler-Einheiten 3 mit ihren Kontaktplatten nach unten hin verschiebbar an der Stange zu befestigen, oder die Strahlereinheiten jeweils an auf die Stange 7 gesteckte Hülsen zu montieren, wobei sich die Hülsen ineinander schieben lassen.

Mittels der erfindungsgemäßen Erwärmungsvorrichtung kann auf schnelle und einfache Weise ein Prothesenschaftrohling erwärmt werden.

## Patentansprüche

1. Erwärmungsvorrichtung für einen thermoplastischen Prothesenschaftrohling, umfassend eine Halterung (1) für den thermoplastischen Prothesenschaftrohling sowie eine Heizeinrichtung, **dadurch gekennzeichnet dass** die Halterung einen Teller (4) zur Aufnahme des Prothesenschaflrohlings (2) sowie einen oder mehrere Strahlungsheizer (3) zur Erwärmung des thermoplastischen Prothesenschaftrohlings (2) aufweist, wobei der Teller (4) drehbar gelagert ist und zum Erwärmen des Prothesenschaftrohlings relativ zu dem oder den feststehenden Strahlungsheizern verdrehbar ist, oder dass der oder die Strahlungsheizer zum Erwärmen des Prothesenschaftrohlings im Inneren des Prothesenschaftrohlings und längs der oder um die Symmetrieachse des Prothesenschaftrohlings angeordnet sind.

2. Erwärmungsvorrichtung nach Anspruch 1, **dadurch gekennzeichnet, dass** bei drehbar gelagertem Teller der oder die Strahlungsheizer (3) innerhalb oder außerhalb des thermoplastischen Prothesenschaftrohlings (2) angeordnet sind.

3. Erwärmungsvorrichtung nach Anspruch 1 oder 2, **dadurch gekennzeichnet, dass** am Teller (4) ein Anschluss (5) für eine Leitung zum Aufblasen des erwärmten thermoplastischen Prothesenschaftrohlings (2) selbst oder eines am Teller (4) angeordneten, den thermoptastischen Prothesenschaftrohling (2) durchsetzenden, ballonartigen Aufblaselements (6) vorgesehen ist

4. Erwärmungsvorrichtung nach einem der vorangehenden Ansprüche,
**dadurch gekennzeichnet, dass** am Teller (4) eine den thermoplastischen Prothesenschaftrohling (2) durchsetzende, vorzugsweise teleskopierbare Stange (7) vorgesehen ist, die am geschlossenen Ende des thermoplastischen Prothesenschaftrohlings (2) austritt und an einem Gestell (8) fixierbar ist.

5. Erwärmungsvorrichtung nach einem der vorangehenden Ansprüche, **dadurch gekennzeichnet, dass** am Teller (4) ein den thermoplastischen Prothesenschaftrohling (2) auch nach Erweichung in seiner Form stabilisierender, wärmeisolierender Stützkörper (9) vorgesehen ist, der vorzugsweise luftdurchlässig ist, vorzugsweise die Strahlung der Strahlungsheizer (3) reflektierend ausgebildet ist, und der vorzugsweise entweder einen den thermoplastischen Prothesenschaftrohling (2) an seinem geschlossenen Ende durchdringenden Stift (10) aufweist, der an einem Gestell (8) fixierbar ist, oder an einer teleskopierbaren Stange (7), die am geschlossenen Ende des thermoplastischen Prothesenschaftrohlings (2) austritt und an einem Gestell (8) fixierbar ist, angeordnet ist.

6. Erwärmungsvorrichtung nach einem der vorangehenden Ansprüche, **dadurch gekennzeichnet, dass** der drehbar gelagerte Teller (4), die am Gestell drehbar fixierbare Stange (7) oder ein den thermoplastischen Prothesenschaftrohling (2) an seinem geschlossenen Ende durchdringender Stift (10) mit einem Motor (11) gekoppelt ist, oder in einer drehgelagerten Aufnahme fixiert ist, die mit einem Motor (11) gekoppelt ist, derart, dass der thermoplastische Prothesenschaftrohling (2) über den Motor (11) bezüglich dem oder den Strahlungsheizern (3) drehbar ist.

7. Erwärmungsvorrichtung nach einem der vorangehenden Ansprüche, **dadurch gekennzeichnet, dass** ein Gestell (8) vorgesehen ist, das einen in seiner Höhe verstellbaren Abschnitt (12) aufweist, an dem die Halterung (1) anbringbar ist.

8. Erwärmungsvorrichtung nach einem der vorangehenden Ansprüche, **dadurch gekennzeichnet, dass** für den oder die Strahlungsheizer (3) ein oder mehrere Abschirmungen (13) vorgesehen sind, die auf ihrer dem Strahler zugewandten Seite bevorzugt für die emittierte Strahlung reflektierend ausgebildet sind.

9. Erwärmungsvorrichtung nach einem der vorangehenden Ansprüche, **dadurch gekennzeichnet, dass** der oder die Strahlungsheizer (3) derart ausgebildet sind, dass ihre Strahlungsleistung zum offenen Ende des Prothesenschaftrohlings (2) hin in einer Weise zunimmt, die trotz differierendem Abstand zwischen Prothesenschaftrohling und Strahlungsheizer ein gleichmäßiges Erwärmen aller zu erwärmenden Bereiche gewährleistet.

10. Erwärmungsvorrichtung nach einem der Ansprüche 1 bis 8, **dadurch gekennzeichnet, dass** die Strahlungsheizer (3) annähernd punktförmig ausgebildet und so längs der Symmetrieachse des Prothesenschaftrohlings (2) aufgereiht und hinsichtlich ihrer Strahlungsleistung gewählt sind, dass sich ihre Strahlung auf dem Prothesenschaftrohling (2) so überlagert, dass trotz differierendem Abstand zwischen Prothesenschaftrohling (2) und den einzelnen Strahlungsheizern (3) ein gleichmäßiges Erwärmen aller zu erwärmenden Bereiche gewährleistet ist.

11. Erwärmungsvorrichtung nach einem der Ansprüche 1 bis 8, **dadurch gekennzeichnet, dass** der oder die Strahlungsheizer (3) bezüglich des Prothesenschafirohlings (2) derart positionierbar sind, dass aufgrund des differierenden Abstands zwischen Prothesenschaftrohling (2) und dem oder den Strahlungsheizern (3) ein gleichmäßiges Erwärmen aller zu erwärmenden Bereiche gewährleistet ist.

12. Erwärmungsvorrichtung nach einem der vorangehenden Ansprüche,
**dadurch gekennzeichnet, dass** ein Strahiungsheizern (3) ein Infrarotstrahler ist.

13. Erwärmungsvorrichtung nach einem der vorangehenden Ansprüche,
**dadurch gekennzeichnet, dass** eine Steuerungseinrichtung (31) vorgesehen ist, der ein Temperatursensor.zum Erfassen der Temperatur des thermoplastischen Prothesenschaftrohling (2) zugeordnet ist, wobei die Steuerungseinrichtung (31) den Betrieb des oder der Strahlungsheizer (3) und/oder des Motors (11) in Abhängigkeit des Sensorerfassungsergebnisses steuert.

14. Erwärmungsvorrichtung nach Anspruch 13, **dadurch gekennzeichnet, dass** in der Steuerungseinrichtung (31) ein oberer und ein unterer Temperaturschwellwert abgelegt ist, wobei die Steuerungseinrichtung (31) den Strahlungsheizer (3) und gegebenenfalls den Motor (11) bei Erfassung einer dem oberen Temperaturschwellwert entsprechenden Temperatur ausschaltet und nach Abkühien bei Erfassung einer dem unteren Temperaturschwellwert entsprechenden Temperatur einschaltet.

15. Erwärmungsvorrichtung nach einem der vorangehenden Ansprüche,
**dadurch gekennzeichnet, dass** der Teller (4) zweiteilig mit einem Außenring und einem in diesen entnehmbar eingesetzten Innenteller ausgeführt ist, wobei der Außenring (14) als Aufnahme für den thermoplastischen Prothesenschaftrohling (2) dient und der Innenteller (15) den oder die Strahlungsheizer (3) trägt.

16. Erwärmungsvorrichtung nach einem der vorangehenden Ansprüche, **dadurch gekennzeichnet, dass** der thermoplastische Prothesenschaftrohling (2) mit Halterung (1) während der Erwärmung formschlüssig von einem ihn nach erreichen der Erweichungstemperatur stützenden wärmeisolierenden Körper (16) aufgenommen ist, wobei der wärmeisolierende Körper (16) vorzugsweise derart ausgebildet ist, dass seine Innenseite die von dem oder den im Inneren des Prothesenschaftrohlings (2) angeordneten Strahlungsheizern (3) abgegebene Heizstrahlung reflektiert.

## Claims

1. A heating device for a thermoplastic prosthesis-socket blank, comprising a holder (1) for the thermoplastic prosthesis-socket blank and a heating device,
**characterised in that**
the holder provides a plate (4) for retaining the prosthesis-socket blank (2) and one or more radiant heaters (3) for heating the.thermoplastic prosthesis-socket blank (2), wherein the plate (4) is mounted in a rotatable manner and can be rotated relative to the fixed radiant heater or heaters in order to heat the prosthesis-socket blank, or that the radiant heater or heaters are arranged in the interior of the prosthesis-socket blank and along or around the axis of symmetry of the prosthesis-socket blank in order to heat the prosthesis-socket blank.

2. The heating device according to claim 1,
**characterised in that**,
with a plate mounted in a rotatable manner, the radiant heater or heaters (3) are arranged inside or outside the thermoplastic prosthesis-socket blank (2).

3. The heating device according to claim 1 or 2,
**characterised in that**,
on the plate (4), a connection (5) is provided for a line for inflating the heated thermoplastic prosthesis-socket blank (2) itself or a balloon-like inflation element (6) inserted through in the thermoplastic prosthesis-socket blank (2) arranged on the plate (4).

4. The heating device according to any one of the preceding claims,
**characterised in that**,
on the plate (4), a preferably telescopic rod (7) inserted through the thermoplastic prosthesis-socket blank (2), which emerges at the closed end of the thermoplastic prosthesis-socket blank (2) and can be fixed to a stand (8), is provided.

5. The heating device according to any one of the preceding claims,
**characterised in that**,
on the plate (4), a thermally insulating support element (9) stabilising the shape of the thermoplastic prosthesis-socket blank (2) even after softening is provided, which is preferably permeable by air and preferably embodied to reflect the radiated heat from the radiant heater (3), and which preferably either provides a pin (10) penetrating the thermoplastic prosthesis-socket blank (2) at its closed end, which can be fixed to a stand, or is arranged on a telescopic rod (7) which emerges at the closed end of the thermoplastic prosthesis-socket blank (2) and can be fixed to a stand (8).

6. The heating device according to any one of the preceding claims,
**characterised in that**
the plate (4) mounted in a rotatable manner, the rod (7) capable of being fixed in a rotatable manner to the stand or a pin (10) penetrating the thermoplastic prosthesis-socket blank (2) at its closed end is connected to a motor (11) or is fixed in a rotatably mounted retainer, which is coupled to a motor (11), in such a manner that the thermoplastic prosthesis-socket blank (2) can be rotated by the motor (11) relative to the radiant heater or heaters (3).

7. The heating device according to any one of the preceding claims,
**characterised in that**
a stand (8), which provides a height-adjustable portion (12) to which the holder (1) can be attached, is provided.

8. The heating device according to any one of the preceding claims,
**characterised in that**
one or more shields (13) are provided for the radiant heater or heaters (3), which are preferably embodied to be reflective for the emitted radiated heat on their side facing towards the radiator.

9. The heating device according to any one of the preceding claims,
**characterised in that**
the radiant heater or heaters (3) are embodied in such a manner that their radiated-heat output increases towards the open end of the prosthesis-socket blank (2) in a manner which guarantees a uniform heating of all of the regions to be heated in spite of the differing spacing distance between the prosthesis-socket blank and the radiant heater.

10. The heating device according to any one of claims 1 to 8,
**characterised in that**
the radiant heaters (3) are embodied in approximately the shape of a spot and arranged in rows along the axis of symmetry of the prosthesis-socket blank (2) and selected with regard to their radiated-heat output in such a manner that their radiated heat overlaps on the prosthesis-socket blank (2) in such a manner that, in spite of the differing spacing distance between the prosthesis-socket blank (2) and the individual radiant heaters (3), a uniform heating of all regions to be heated is guaranteed.

11. The heating device according to any one of claims 1 to 8,
**characterised in that**
the radiant heater or heaters (3) can be positioned relative to the prosthesis-socket blank (2) in such a manner that a uniform heating of all regions to be heated is guaranteed because of the differing spacing distance between the prosthesis-socket blank (2) and the radiant heater or heaters (3).

12. The heating device according to any one of the preceding claims,
**characterised in that**
a radiant heaters (3) is an infrared spotlight.

13. The heating device according to any one of the preceding claims,
**characterised in that**
a control device (31) to which a temperature sensor is allocated for registering the temperature of the thermoplastic prosthesis-socket blank (2) is provided, wherein the control device (31) controls the operation of the radiant heater or heaters (3) and/or the motor (11) dependent upon the result registered by the sensor.

14. The heating device according to claim 13,
**characterised in that**
an upper and lower temperature-threshold value is stored in the control device (31), wherein the control device (31) switches off the radiant heater (3) and optionally the motor (11) when it registers a temperature corresponding to the upper temperature threshold, and switches it on after cooling, when it registers a temperature corresponding to the lower temperature threshold.

15. The heating device according to any one of the preceding claims,
**characterised in that**
the plate (4) is embodied in two parts with an outer ring and an inner plate fitted in a detachable manner into the latter, wherein the outer ring (14) is used as a retainer for the thermoplastic prosthesis-socket blank (2), and the inner plate (15) bears the radiant heater or heaters (3).

16. The heating device according to any one of the preceding claims,
**characterised in that**
the thermoplastic prosthesis-socket blank (2) with holder (1) is retained in a form-fit manner during the heating by a thermally insulating element (16) which supports it when it reaches the softening temperature, wherein the thermally insulating element (16) is preferably embodied in such a manner that its inner side reflects the heat radiated from the radiant heater or heaters (3) arranged in the interior of the prosthesis-socket blank (2).

## Revendications

1. Dispositif de chauffage pour une ébauche de prothèse thermoplastique, comprenant un support (1) pour l'ébauche de prothèse thermoplastique ainsi qu'un dispositif de chauffage, **caractérisé en ce que** le support comprend un plateau (4) destiné à recevoir l'ébauche de prothèse (2) ainsi qu'un ou plusieurs radiateurs à rayonnement (3) destinés à réchauffer l'ébauche de prothèse thermoplastique (2), étant entendu que le plateau (4) est monté de façon rotative et peut tourner par rapport au ou aux radiateurs à rayonnement fixes afin de réchauffer l'ébauche de prothèse, ou **en ce que** le ou les radiateurs à rayonnement destinés à réchauffer l'ébauche de prothèse sont agencés à l'intérieur de l'ébauche de prothèse et le long ou autour de l'axe de symétrie de l'ébauche de prothèse.

2. Dispositif de chauffage selon la revendication 1, **caractérisé en ce que** dans le cas d'un plateau monté de façon rotative, le ou les radiateurs à rayonnement (3) sont agencés à l'intérieur ou à l'extérieur de l'ébauche de prothèse thermoplastique (2).

3. Dispositif de chauffage selon la revendication 1 ou 2, **caractérisé en ce qu'**il est prévu au niveau du plateau (4) un raccordement (5) pour une conduite destinée à souffler l'ébauche de prothèse thermoplastique (2) chauffée elle-même ou un élément de gonflage (6) du type d'un ballon traversant l'ébauche de prothèse thermoplastique (2) et agencé sur le plateau (4).

4. Dispositif de chauffage selon l'une quelconque des revendications précédentes, **caractérisé en ce qu'**il est prévu au niveau du plateau (4) une tige (7) de préférence télescopique, traversant l'ébauche de prothèse thermoplastique (2), qui dépasse au niveau de l'extrémité fermée de l'ébauche de prothèse thermoplastique (2) et peut être fixée à un bâti (8).

5. Dispositif de chauffage selon l'une quelconque des revendications précédentes, **caractérisé en ce qu'**il est prévu au niveau du plateau (4) un élément d'appui (9) isolant à la chaleur stabilisant l'ébauche de prothèse thermoplastique (2) dans sa forme y compris après le ramollissement, qui est de préférence perméable à l'air, qui est de préférence réalisé de façon à réfléchir le rayonnement des radiateurs à rayonnement (3) et qui de préférence soit comprend une broche (10) passant à travers l'ébauche de prothèse thermoplastique (2) au niveau de son extrémité fermée, qui peut être fixée à un bâti (8), soit est agencé sur une tige télescopique (7) qui dépasse au niveau de l'extrémité fermée de l'ébauche de prothèse thermoplastique (2) et peut être fixée à un bâti (8).

6. Dispositif de chauffage selon l'une quelconque des revendications précédentes, **caractérisé en ce que** le plateau (4) monté de façon rotative, la tige (7) pouvant être fixée de façon rotative sur le bâti ou une broche (10) passant à travers l'ébauche de prothèse thermoplastique (2) au niveau de son extrémité fermée est couplé avec un moteur (11) ou est fixé dans un logement monté en rotation qui est couplé avec un moteur (11) de telle sorte qu'il est possible de faire tourner l'ébauche de prothèse thermoplastique (2) par rapport au ou aux radiateurs à rayonnement (3) au moyen du moteur (11).

7. Dispositif de chauffage selon l'une quelconque des revendications précédentes, **caractérisé en ce qu'**il est prévu un bâti (8) qui comprend une section (12) réglable dans sa hauteur sur laquelle le support (1) peut être installé.

8. Dispositif de chauffage selon l'une quelconque des revendications précédentes, **caractérisé en ce qu'**il est prévu pour le ou les radiateurs à rayonnement (3) un ou plusieurs blindages (13) qui sont de préférence réalisés, de leur côté dirigé vers le radiateur, de façon à réfléchir le rayonnement émis.

9. Dispositif de chauffage selon l'une quelconque des revendications précédentes, **caractérisé en ce que** le ou les radiateurs à rayonnement (3) sont réalisés de telle sorte que leur puissance de rayonnement augmente en direction de l'extrémité ouverte de l'ébauche de prothèse (2) d'une manière qui garantit un chauffage uniforme de toutes les zones à chauffer malgré la distance différente entre l'ébauche de prothèse et les radiateurs à rayonnement.

10. Dispositif de chauffage selon l'une quelconque des revendications 1 à 8, **caractérisé en ce que** les radiateurs à rayonnement (3) sont réalisés sensiblement en forme de points et qu'ils sont alignés le long de l'axe de symétrie de l'ébauche de prothèse (2) et choisis au niveau de leur puissance de rayonnement de telle sorte que leur rayonnement se superpose à l'ébauche de prothèse (2) de telle sorte qu'un chauffage uniforme de toutes les zones à chauffer est garanti malgré la distance différente entre l'ébauche de prothèse (2) et les radiateurs à rayonnement (3) individuels.

11. Dispositif de chauffage selon l'une quelconque des revendications 1 à 8, **caractérisé en ce que** le ou les radiateurs à rayonnement (3) peuvent être positionnés par rapport à l'ébauche de prothèse (2) de telle sorte qu'un chauffage uniforme de toutes les zones à chauffer est garanti malgré la distance différente entre l'ébauche de prothèse (2) et le ou les radiateurs à rayonnement (3).

12. Dispositif de chauffage selon l'une quelconque des revendications précédentes, **caractérisé en ce qu'**un radiateur à rayonnement (3) est un radiateur infrarouge.

13. Dispositif de chauffage selon l'une quelconque des revendications précédentes, **caractérisé en ce qu'**il est prévu un dispositif de commande (31), auquel est adjoint un capteur de température destiné à mesurer la température de l'ébauche de prothèse thermoplastique (2), étant entendu que le dispositif de commande (31) commande le fonctionnement du ou des radiateurs à rayonnement (3) et/ou du moteur (11) en fonction du résultat de la mesure du capteur.

14. Dispositif de chauffage selon la revendication 13, **caractérisé en ce qu'**une valeur seuil supérieure et inférieure de la température est définie dans le dispositif de commande (31), étant entendu que le dispositif de commande (31) arrête le radiateur à rayonnement (3), et le cas échéant, le moteur (11) en cas de mesure d'une température correspondant à la valeur seuil supérieure de la température et les met en marche après un refroidissement en cas de mesure d'une température correspondant à la valeur seuil inférieure de la température.

15. Dispositif de chauffage selon l'une quelconque des revendications précédentes, **caractérisé en ce que** le plateau (4) est réalisé en deux parties avec un cercle extérieur et un plateau intérieur logé dans celui-ci de façon amovible, étant entendu que le cercle extérieur (14) fait fonction de support pour l'ébauche de prothèse thermoplastique (2) et que le plateau intérieur (15) supporte le ou les radiateurs à rayonnement (3).

16. Dispositif de chauffage selon l'une quelconque des revendications précédentes, **caractérisé en ce que** l'ébauche de prothèse thermoplastique (2) est reçue avec le support (1), pendant le chauffage, par engagement mécanique dans un élément isolant à la chaleur (16) qui la supporte lorsque la température de ramollissement est atteinte, étant entendu que l'élément isolant à la chaleur (16) est de préférence réalisé de telle sorte que son côté intérieur réfléchit le rayonnement chauffant émis par le ou les radiateurs à rayonnement (3) agencés à l'intérieur de l'ébauche de prothèse (2).
